# EUROPÄISCHE PATENTSCHRIFT

(11) **EP 0 821 060 B1**
(45) Veröffentlichungstag und Bekanntmachung des Hinweises auf die Patenterteilung: **06.04.2005**
(21) Anmeldenummer: 97112623.0
(22) Anmeldetag: 23.07.1997
(51) Int. Cl.: C12N 15/12, C07K 14/705, C07K 1/06, C07K 16/30, C12Q 1/68, C12N 5/10, C12N 5/20, G01N 33/574, A61K 39/395, A01K 67/027

(54) **E-Cadherin-Mutationen als Grundlage zur Diagnostik und Therapie humaner maligner Tumoren**
Diagnosis and therapy of human malign tumors based on E-cadherin mutations
Diagnostic et thérapie des tumeurs malignes humaines basés sur des mutations d'E-cadherin

(30) Priorität: 24.07.1996 DE 19629938
(43) Veröffentlichungstag der Anmeldung: 28.01.1998
(73) Patentinhaber: GSF-Forschungszentrum für Umwelt und Gesundheit GmbH, 85764 Oberschleissheim (DE)
(72) Erfinder: Höfler, Heinz, Prof. Dr., 81675 München (DE); Becker, Karl-Friedrich, Dr., 85748 Garching b. München (DE); Kremmer, Elisabeth, Dr., 85354 Freising (DE); Eulitz, Manfred, Dr., 81677 München (DE); Schuhmacher, Christoph, Dr., 80639 München (DE)
(74) Vertreter: Reinhard - Skuhra - Weise & Partner

(56) Entgegenhaltungen:
- WO-A-94/11401
- DE-A- 4 110 405
- H. HÖFLER ET AL.: "Gastric carcinogenesis. New strategies of diagnosis and therapyon the basis of molecular biology." 1996 , MONDUZZI EDITORE , BOLOGNA, ITALIEN XP000979466 * Seite 93 - Seite 97 *
- K. BECKER ET AL.: "E-cadherin gene mutations provide clues to diffuse type gastric carcinomas." CANCER RESEARCH, Bd. 54, Nr. 14, 15. Juli 1994 (1994-07-15), Seiten 3845-3852, XP002159873 Baltimore, MD, VSA
- C. SCHUHMACHER ET AL.: "E-cadherin mutation specific antibody in gastric cancer: proposal for a multi-center study to open novel clinical avenues." In: Progress in gastric research, Band 1, Eds. J. Siewert et al." 1997 , MONDUZZI EDITORE , BOLOGNA, ITALIEN XP000979476 * Seite 427 - Seite 431 *
- H. HÖFLER ET AL.: "Gastric carcinogenesis: new strategies of diagnosis and therapy on the basis of molecular biology." In: Progress in gastric research, Band 1, Eds. J. Siewert et al." 1997 , MONDUZZI EDITORE , BOLOGNA, ITALIEN XP000979462 * Seite 393 - Seite 398 *

## Beschreibung

Die Erfindung betrifft monoklonale Antikörper, die spezifisch gegen mutiertes, transmembranständiges E-Cadherin gerichtet sind und die zum Nachweis von Magenkarzinomen einsetzbar sind.

### Einleitung

Maligne Tumoren stehen weltweit in der Statistik der Todesursachen an vorderer Stelle. Das Magenkarzinom ist hierbei international der zweithäufigste Tumor mit Todesfolge. Im Zusammenhang mit dem Magenkarzinom sind verschiedene genetische Alterationen beschrieben worden. Diese beinhalten Mikrosatelliten-Instabilitäten und Veränderungen der Gene p53, APC DCC (Tahara E: Genetic alterations in human gastrointestinal cancers. Cancer 1995; 75: 1410-1417). Histomorphologisch kann man beim Magenkarzinom zwei Typen unterscheiden (Laurén P: The two histological main types of gastric carcinoma: diffuse and so-called intestinal-type of carcinoma. Acta Pathol Microbiol Scand 1965; 64: 31-49): einmal intestinale Karzinome mit drüsiger Differenzierung und zum anderen diffuse Karzinome mit aufgehobener Gewebearchitektur. Der genetische Grund für diese zweigeteilte Entwicklung und Morphologie war bislang nicht geklärt. Hierzu könnten Veränderungen des E-Cadherin Moleküls möglicherweise von Bedeutung sein. E-Cadherin ist ein homophiles, transmembranständiges Zelladhäsionsmolekül, welches bei der Interaktion von epithelialem Gewebe eine wesentliche Rolle spielt. Erste molekularbiologische Studien am 16 Exon umfassenden E-Cadherin Gen wiesen darauf hin, daß Mutationen zur Morphologie und zum Wachstumstyp von Magenkarzinomen beitragen könnten (Becker K-F, Atkinson MJ, Reich U, Becker I, Nekarda H, Siewert JR, Höfler H: E-Cadherin gene mutations provide clues to diffuse type gastric carcinomas. Cancer Res 1994; 54(14): 3845-3852).

Grundsätzlich sind Veränderungen in malignen Tumoren zur Erklärung eines bestimmten biologischen Verhaltensmusters interessant; diese Phänomene sind als spezifisches Charakteristikum und damit als Tumormarker verwertbar. Hierzu zählen Zellprodukte wie auch typische Zelloberflächeneigenschaften, die direkt oder indirekt nachgewiesen werden können. Bisher ist es jedoch nicht gelungen, ein ausschließlich auf Tumorzellen beschränktes Oberflächenantigen oder Zellprodukt zu isolieren. Als Grundlage für Diagnostik und Therapie maligner Erkrankungen wird bislang das vermehrte Auftreten bestimmter Zelleigenschaften (Oberflächenantigen, intrazelluläre Proteine, sezernierte Zellprodukte) im Körper in Relation zu gesundem Gewebe genutzt.

Bisheriger Stand der diagnostischen und therapeutischen Nutzung von E-Cadherin:

In ersten Publikationen wurde berichtet, daß E-Cadherin - immunhistochemisch mittels spezifischer Antikörper nachgewiesen - bei Tumorzellen ein verändertes Expressionsmuster aufwies. Tumorgewebe zeigte teilweise eine reduzierte oder fehlende E-Cadherin Immunreaktivität (s. Tab. 1). Diese Tatsache wurde von anderen Autoren als Ursache einer verminderten Produktion ("downregulation") des Proteins im Tumor angenommen (vgl. Birchmeier, DE-A-41 10 405 A1).

**Tabelle 1.**

| E-Cadherin Immunreaktivität in Magenkarzinom-Patienten. | | | | |
|---|---|---|---|---|
| | | E-Cadherin Immunreaktivität | | |
| Histologie | n^{a} | unverändert^{b} | abnormal^{c} | Referenz |
| | | | | |
| diffus | 28 | 13 (46%) | 15 (54%) | 1 |
| intestinal | 93 | 69 (74%) | 24 (26) | 1 |
| | | | | |
| diffus | 14 | 7 (50%) | 7 (50%) | 2 |
| intestinal | 22 | 21 (95%) | 1 (5%) | 2 |
| | | | | |
| diffus | 11 | 6 (55%) | 5 (45%) | 3 |
| | | | | |
| diffus | 21 | 0 | 21 (100%) | 4 |
| intestinal | 30 | 5 (17%) | 25 (83%) | 4 |
| | | | | |
| diffus | 27 | 19 (70%) | 8 (30%) | 5 |
| intestinal | 17 | 17 (100%) | 0 | 5 |

Legende zur Tabelle 1: ^{a}Anzahl der untersuchten Patienten; ^{b}E-Cadherin Immunreaktivität ähnlich zu der im "normalen" Gewebe; ^{c}Verminderte oder keine E-Cadherin Immunreaktivität im Tumor.

Referenzen zur Tabelle 1.
1 Shino Y, Watanabe A, Yamada Y, Tanase M, Yamada T, Matsuda M, Yamashita J, Tatsumi M, Miwa T, Nakano H: Clinicopathologic evaluation of immunohistochemical E-Cadherin Expression in human gastric carcinomas. Cancer 1995; 76: 2193-2201.
2 Brito MJ, Jacinto L, Jankowski J, Pignatelli M, Filipe MI: E-Cadherin (cell adhesion molecule) in gastric carcinoma. Path.Res.Pract. 1995; 191:628 [Abstract].
3 Matsui S, Shiozaki H, Inoue M, Tamura S, Doki Y, Kadowaki T, Iwazawa T, Shimaya K, Nagafuchi A, Tsukita S, Mori T: Immunohistochemical evaluation of alpha-catenin expression in human gastric cancer. Virchows Archiv 1994; 424: 375-381.
4 Mayer B, Johnson JP, Leitl F, Jauch KW, Heiss MM, Schildberg FW, Birchmeier W, Funke I: E-Cadherin expression in primary and metastatic gastric cancer: down-regulation correlates with cellular dedifferention and glandular disintegration. Cancer Res 1993;53:1690-1695.
5 Shimoyama Y, Hirohashi S: Expression of E- and P-cadherin in gastric carcinomas. Cancer Res 1991;51(8):2185-2192.

Eigene Überlegungen hinsichtlich dieser Phänomene zielten erstmals auf die Integrität des E-Cadherin Gens. Die molekularbiologische Untersuchung von Magenkarzinomgewebe zeigte nach RNA-Extraktion, reverser Transkription und direkter cDNA-Sequenzierung Defekte im E-Cadherin Gen. Magenkarzinome des diffusen Subtyps wurden in einem Teilbereich des E-Cadherin Gens (Exon 6-10 und 13-16) auf genetische Alterationen untersucht. Beobachtet wurden Verluste der Exons 8 und 9, ein Teilverlust des Exons 10, bzw. eine Punktmutation im Bereich des Exons 8 (Becker K-F, Atkinson MJ, Reich U, Becker I, Nekarda H, Siewert JR, Höfler H: E-Cadherin gene mutations provide clues to diffuse type gastric carcinomas. Cancer Res 1994; 54(14): 3845-3852). Tumoren des intestinalen Subtyps wiesen keine strukturverändernden Mutationen auf. Die Analyse der gefundenen Mutationen zeigte, daß gewisse Häufungen einzelner Deletionen auftraten, jedoch auch Punktmutationen oder kleinere Deletionen zu beobachten waren. Die immunhistochemische Färbung (Antikörper: HECD-1, Takara Biomedicals, Japan, vgl. Methodik) von einigen der Fälle mit mutiertem E-Cadherin zeigte eine überwiegend membranständige Färbung der Tumorzellen wie auch eine Färbung nicht tumoröser Mucosa. Es konnte jedoch nicht unterschieden werden, ob es sich bei der Tumorzell-Markierung um den Nachweis von Wildtyp E-Cadherin oder mutiertem E-Cadherin handelte. Zum einen bestand die Möglichkeit, daß mutiertes Protein weiterhin in der Zellmembran inkorporiert wurde und der E-Cadherin Antikörper (HECD-1) ein Epitop außerhalb der mutierten Region erkennt. Eine weitere Erklärung wäre die Bindung des Antikörpers an Wildtyp-E-Cadherin, welches - generiert durch das nicht mutierte, zweite Allel - ebenfalls in Tumorzellen exprimiert wird. Die Tatsache, daß einige der Tumoren keine Färbung aufwiesen, ließ zunächst auf weitere Mutationen außerhalb der untersuchten Exons 6-10 und 13-16 schließen, die möglicherweise Einfluß auf die Translation oder die Stabilität des Proteins haben.

Es ist eine Aufgabe der vorliegenden Erfindung, monoklonale Antikörper bereitzustellen, die sich spezifisch zum Nachweis von Magenkarzinomen, insbesondere diffusen Magenkarzinomen, eignen.

Diese Aufgabe wird erfindungsgemäß durch die im Anspruch 1 näher gekennzeichneten monoklonalen Antikörper gelöst. Bevorzugte Ausführungsformen der Erfindung ergeben sich aus den Unteransprüchen sowie aus den nebengeordneten Ansprüchen.

Die Analyse bislang nicht untersuchter Abschnitte des E-Cadherin Gens (Exons 1-5 , 11 und 12), wie auch die Sequenzierung der E-Cadherin cDNA von 10 weiteren Magenkarzinomfällen, ergaben erneut Mutationen bei diffusen Magenkarzinomen (vgl. den Abschnitt "Methodik"). Diese neu entdeckten Mutationen (Tabelle 2) zeigten überraschenderweise weiterhin ein typisches Muster. In allen Fällen handelt es sich entweder um Multimere eines Basentripletts, die den Leserahmen nicht beeinträchtigen, oder um Punktmutationen. Die Annahme nach der immunhistochemischen Untersuchung, daß der Verlust der Immunreaktivität aufgrund von translations-unterbrechenden Mutationen herbeigeführt werden könnte, ließ sich aufgrund dieser Untersuchungen ausschließen. Parallel zu den Untersuchungen an Magenkarzinomen wurden andere epitheliale Tumoren analysiert. Gewebe von Mammakarzinomen, Ösophaguskarzinomen und Dickdarmkarzinomen wiesen in keinem der Fälle dem Magenkarzinom entsprechende Mutationen auf.

**Tabelle 2**

| Von uns gefundene E-Cadherin Mutationen in Magenkarzinomen. | | | | |
|---|---|---|---|---|
| **Mutation** | **Exon** | **Nukleotidposition** | **Aminosäure(n)** | **Mutationsart** |
| 563del63 | 4 | 563 | Aminosäuren 157-177 | in frame-Deletion von 63 bp |
| 706del9 | 5 | 706 | 205-207 | in frame-Deletion von 9 bp |
| 826del9 | 6 | 826 (T zu G) | 245-247; 244 (Asp zu Glu) | in frame-Deletion von 9 bp und Missense Mutation |
| 1036del15 | 7 | 1036 | 315-319 | in frame-Deletion von 15 bp |
| 1103del129 | 8 | 1103 | 337-379 | in frame-Deletion von 129 bp (Verlust des kompletten Exons 8) |
| Asp370Ala | 8 | 1203 | 370 (Asp zu Ala) | Missense Mutation |
| 1232del183 | 9 | 1232 | 380-440 | in frame-Deletion von 183 bp (Verlust des kompletten Exons 9) |
| 1414del69 | 10 | 1414 | 441-463 | in frame-Deletion von 69 bp |
| Val473Asp | 10 | 1512 | 473 (Val zu Asp) | Missense Mutation |
| Arg598Gln | 12 | 1887 | 598 (Arg zu Gln) | Missense Mutation |

Die zusätzlich durchgeführten Untersuchungen über die Charakteristik der Mutationen, sowie die Sequenzierung weiterer Tumoren, führen nunmehr zu einem bislang nicht beschriebenen und für Karzinome einzigartigen Prinzip: E-Cadherin Alterationen im diffusen Magenkarzinom sind in-frame Mutationen (keine Unterbrechung des Leserasters). Dieses Ergebnis ist für diagnostische und therapeutische Zwecke einsetzbar.

Die Erfindung wird nachfolgend anhand von Abbildungen näher erläutert. Die Abbildungen zeigen:
- Abb. 1: eine Übersicht über die bisher gefundenen E-Cadherin-Mutationen in diffusen Magenkarzinomen, die typischerweise In-frame-Deletionen darstellen;
- Abb. 2: den Nachweis der membranständigen Lokalisation von mutiertem E-Cadherin durch Immunfluoreszenz;
- Abb. 3: den Nachweis der membranständigen Lokalisation von mutiertem E-Cadherin durch Immun-Elektronenmikroskopie.
- Abb. 4: Fließschema zur Schnelldiagnostik von E-Cadherin-Mutationen gemäß der Erfindungen.
- Abb. 5: Western-Blot mit mutations-spezifischem E-Cadherin Antikörper 7E6.
- Abb. 6: Immunhistochemie mit mutations-spezifischem E-Cadherin Antikörper 7E6.

### Beschreibung der Erfindung

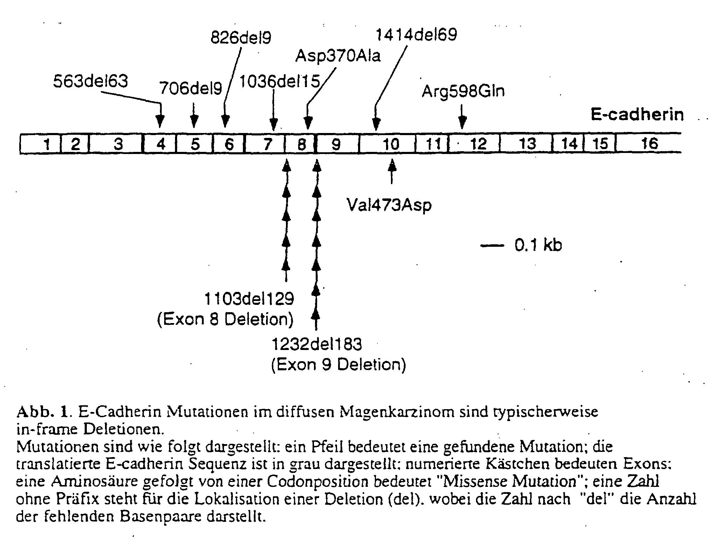

Es ist gelungen, für das diffuse Magenkarzinom eine typische Mutationsform - in frame Mutationen (Deletionen von Multimeren eines Basentripletts bzw. Punktmutationen) - nachzuweisen (Abb. 1).

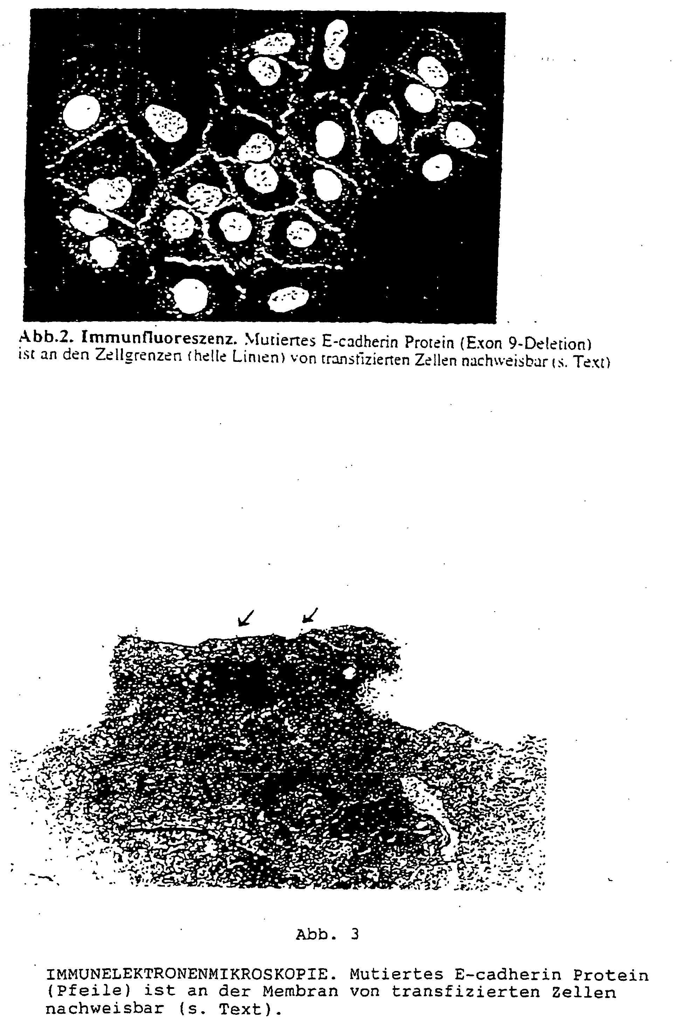

Diese charakteristischen Mutationen lassen den Schluß zu, daß in allen Fällen ein verändertes Protein generiert und weiterhin von der Zelle exprimiert wird. Der Nachweis dieser Vermutung wurde durch Transfektion des mutierten Gens in eine E-Cadherin freie Zellinie (MDA-Mammakarzinomzellen) geführt (Methodik siehe Anhang). Stabil transfizierte Zellen exprimierten das mutierte Protein an der Zellmembran. Der Nachweis der membranständigen Lokalisation des mutierten Proteins wurde durch Immunfluoreszenz (Abb. 2) und Elektronenmikroskopie (Abb.3) am Beispiel der Exon 9-Deletion zweifelsfrei geführt. Die ungestörte Membranständigkeit des mutierten Proteins ist für die weitere Verwertung in Diagnostik und Therapie von entscheidender Bedeutung.

Der Verlust von Basensequenzen, bzw. das Auftreten von Punktmutationen läßt bei aufrechterhaltener Transkription eine neue, einzigartige und unverwechselbare RNA-Sequenz entstehen. Dieser "Fehler" wird dann bei der weiteren Translation durch eine veränderte Aminosäurensequenz im Protein festgehalten. Die durch die Mutationen neu entstandenen Proteinsequenzen sind in der Tabelle 3 im einzelnen aufgeführt.

Die einzelnen Tumorzellen sind durch diese Sequenzen , d.h. über ihr mutiertes E-Cadherin Protein, unverwechselbar markiert. Eine durchgeführte Recherche (Homologievergleich) in mehreren Gendatenbanken (via EMBL/GenBank / SWISS-PROT / PDB, Release 32.0) ergab keine Ähnlichkeit zu bisher bekannten Sequenzen. Zur Frage der Klonalität des Mutations-Ereignisses wurden Magenfrühkarzinome untersucht. Da auch hierbei Mutationen nachweisbar waren (eigene Beobachtungen), müssen E-Cadherinmutationen ein frühes Ereignis in der Tumorentstehung sein, wahrscheinlich von dem malignen Ursprungsklon ausgehend.

**Tabelle 3**

| Durch Mutationen neu entstandene E-Cadherin Proteinsequenzen. | | |
|---|---|---|
| **Mutation** | **"Normale" E-Cadherin Sequenz** | **Neue E-Cadherin Sequenz** |
| 563del63 | PGLRRQKRDW/VIPPISCPEN | PGLRRQKRDW/IKSNKDKEGK |
| 706del9 | QGADTPPVGV/FIIERETGWL | QGADTPPVGV/ERETGWLKVT |
| 1036del15 | LSQDPELPDK/NMFTINRNTG | LSQDPELPDK/NRNTGVISVV |
| 1103del129 | SVVTTGLDRE/SFPTYTLVVQ | SVVTTGLDRE/YKGQVPENEA |
| 1232del183 | DNPPIFNPTT/YKGQVPENEA | DNPPIFNPTT/GLDFEAKQQY |
| 1414del69 | NNDGILKTAK/GLDFEAKQQY | NNDGILKTAK/VSLTTSTATV |
| Asp370Ala | TAVITVTDTN**D**NPPIFNPTT | TAVITVTDTN**A**NPPIFNPTT |
| Val473Asp | EVSLTTSTAT**V**TVDVLDVNE | EVSLTTSTAT**D**TVDVLDVNE |
| Arg598Gln | VNDNAPIPEP**R**TIFFCERNP | VNDNAPIPEP**Q**TIFFCERNP |
| 826del9 | AVSSNGNAVE**D**/PMEILITV | AVSSNGNAVE**E**/ILITVTDQN |

Der Schrägstrich (/) zeigt die Position einer Deletion an; ab dieser Stelle ändert sich die Proteinsequenz; unterstrichene und fettgedruckte Buchstaben markieren durch Punktmutationen veränderte Aminosäuren.

Die in Tabelle 3 gezeigten und weitere, noch unbekannte E-Cadherin Mutationen lassen sich beispielsweise wie folgt für diagnostische und therapeutische Fragestellungen nutzen:

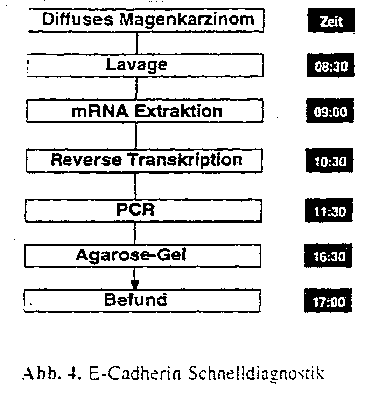

RNA Schnellextraktion und nachfolgender PCR-Nachweis der Mutationen:
Die Charakteristik der beschriebenen Mutationen (hauptsächlich Deletionen!) eignet sich hervorragend zu einem gezielten Schnelltest: die Amplifikation eines cDNA-Abschnittes durch entsprechende Primer (s. Methodik), der die mutierten Exonbereiche einschließt - in Kombination mit einer RNA-Schnellextraktion (s. Methodik) - erlaubt die klinische Nutzbarmachung der Erfindung (Zeitfaktor!). Der Nachweis kann hierbei in Geweben (z.B. Biopsien, Punktaten, Zytologien) und Körperflüssigkeiten (z.B. Blut) geführt werden.
Beispielhaft wurden Biopsien und Peritoneallavagen (Bauchfellspülungen) von Magenkarzinompatienten auf Mutationen des E-Cadherin Gens untersucht. Innerhalb von 8 Stunden (s. Abb. 4) ist es möglich, eine E-Cadherin Alteration spezifisch nachzuweisen und damit für klinische Fragestellungen zeitgerechte Informationen zu liefern. Im Zusammenhang mit den weiteren Ausführungen (s. Antikörper/Therapie) kann hierdurch rasch auf den Behandlungsplan Einfluß genommen werden. Grundsätzlich kann diese Methodik zum spezifischen Erstnachweis eines Magenkarzinoms eingesetzt werden. Auch die Verwendung zur Differentialdiagnose (z.B. Metastasenbiopsie) zu anderen Malignomen bei unbekanntem Primärtumor (z.B. Mammakarzinomen) ist möglich.

### Antikörper

Die Kombination von Membranständigkeit und Tumorspezifität des mutierten E-Cadherin Proteins erlaubte erstmals die spezifische Produktion eines ausschließlich gegen Tumorzellen gerichteten monoklonalen Antikörpers.

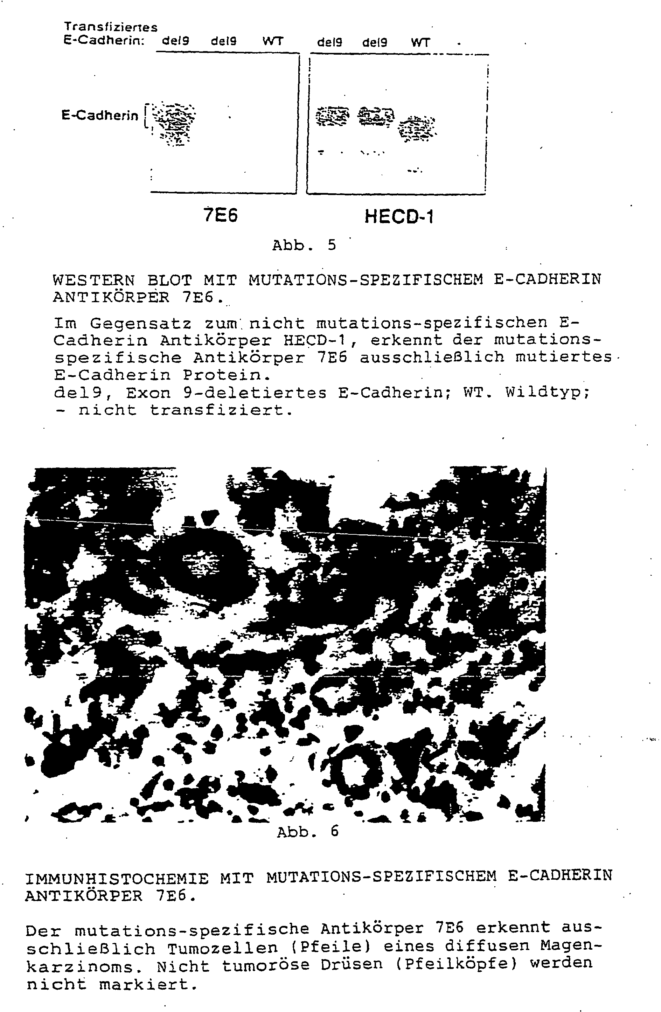

Durch die erfindungsgemäß bereitgestellten, tumor-spezifischen Gensequenzen ist erstmals die Möglichkeit gegeben, gezielt Aminosäuren zu benennen, deren Sequenz ein entsprechendes, ausschließlich auf Tumorzellen befindliches Protein beschreibt. Hierdurch wird die Generierung von Antikörpen erreicht, die sich selektiv gegen die mutierte E-Cadherin Region richten. Zur Bestätigung dieser Hypothese wurde von uns über die neu entstandene Sequenz am Beispiel der Exon 9-Deletion, beispielhaft die Herstellung eines monoklonalen Antikörpers gegen das verkürzte Transmembranprotein versucht (Methodik im Anhang). Unklar war hierbei, ob sich der mutierte Bereich als antigene Determinante eignen würde, da die sterische Anordung des Epitops nicht bekannt ist. Es wurden 23 Hybridome (Antikörperproduzierende Klone) generiert und deren Spezifität in vitro getestet (s. Anhang). Ein Klon (7E6-1) wurde gefunden, der sowohl im Western-Blot und in der Immunfluoreszenz ausschließlich Exon 9 deletiertes E-Cadherinprotein erkennt. "Normales" E-Cadherin wird vom 7E6 Antikörper nicht detektiert (Abb. 5). Diese den 7E6-Antikörper produzierende Zellinie wurde bei der DSM hinterlegt (Eingangsnr. DSM ACC 2277) (Bezugszeichen: delta CAD-9, clone 7E6-1). Der 7E6 Antikörper erkennt die Mutation 1232del183.

Nicht vorhersehbar war die Funktionsfähigkeit dieses Antikörpers an Formalin fixiertem und in Paraffin eingebettetem Material. Erstmals konnten hierdurch mittels immunhistochemischer Methoden spezifisch Tumorzellen auf einem histologischen Schnitt nachgewiesen werden (Abb. 6). Nicht tumoröse Zellen auf dem selben Schnitt wurden nicht markiert! Hierdurch ist die Zuordnung des mutierten Moleküls zu einzelnen Zellen ermöglicht. Ein vergleichbar spezifischer Tumormarker ist nach unserer Kentnis bisher nicht beschrieben worden.

Die bei der DSM unter der Eingangsnr. ACC 2277 hinterlegte Hybridomzellinie ist nur ein Beispiel für eine Zellinie, die die erfindungsgemäß bereitgestellten monoklonalen Antikörper produziert. Erfindungsgemäß wurden bereits weitere Antikörper hergestellt, die die in frame-Mutationen erkennen. Die Bereitstellung dieser Antikörper liegt im Bereich des Fachkönnens des auf diesem Gebiet arbeitenden Fachmanns.

Der 7E6 monoklonale Antikörper, wie auch alle weiteren generierten Antikörper, die sich mutationsspezifisch gegen E-Cadherin richten, können wie nachfolgend beschrieben diagnostisch und therapeutisch eingesetzt werden.

Diagnostik mittels mutations-spezifischer E-Cadherin Antikörper: Retrospektive Untersuchung von Formalin fixiertem und Paraffin eingebettetem Gewebe auf bestimmte E-Cadherin Defekte mittels Immunhistochemie.

Prospektive Untersuchung
in vitro von
- Blut zum Nachweis zirkulierender Tumorzellen: als Basisscreening auf diffuses Magen CA zur Beurteilung der prä-, intra- und postoperativen Tumorzelldissemination als möglicher Krankheitsverlaufsmarker
- Tumorbiopsie: zum prätherapeutischen Nachweis mutierten E-Cadherins im Hinblick auf Planung einer präoperativen (neoadjuvanten), intraoperativen (Peritoneum, Pfortader) und postoperativen (additiven oder adjuvanten) Therapie
- OP Präparat: zum Nachweis von Tumorzellen (z.B. Abtragungsrand, Sicherung des Resektionsausmaßes)
- Lymphknoten: zum Nachweis von Metastasen und Tumorzellen ( "Microenvolvement")
- Lavage: zum Nachweis von disseminierten Tumorzellen in der Bauchhöhle
- Gewebe: zum Nachweis von disseminierten Tumorzellen (Immunhistochemie)
- Differentialdiagnose von Karzinomen: Nachweis von charakteristischen E-Cadherin Mutationen über Immunhistochemie zur differentialdiagnostischen Beurteilung von verdächtigen Gewebsproben
- Screening von Hochrisikogruppen ("Krebsfamilien"): Nachweis von E-Cadherin Mutationen in Blutzellen (DNA)
- in vivo: Grundvoraussetzung: Humanisierung des Antikörpers zur therapeutischen Anwendung
spezifischer Nachweis von Tumorzellen mittels Immunszintigraphie:
prätherapeutisch zur Operationsplanung/Therapieplanung
zur Verlaufskontrolle von Therapie bzw.
zur Bestätigung eines Therapieerfolges und zur posttherapeutischen Verlaufskontrolle

Therapie mittels mutations-spezifischer E-Cadherin Antikörper:
- Immunradiotherapie: Koppelung einer Strahlenquelle an den Mutationsspezifischen Antikörper
- Immuntoxintherapie: Koppelung eines Toxins (z.B. Pseudomonastoxin) an den Antikörper

Somatische Gentherapie mittels mutations-spezifischer E-Cadherin Antikörper:
- Mögliche Wege eines spezifischen Gentransfers:: Koppelung mit viralen Genexpressionssystemen (z.B. Adenoviren, oder MVA, Vaccinia-abgeleitete Expressionsvektoren);
Koppelung mit nicht-viralen Genexpressionssystemen (z.B. T7 RNA Polymerase + T7 DNA Vektor);
- Möglicher gentechnischer Therapieansatz:: Einbringen von Cofaktoren (z.B. B7) zur Markierung der Tumorzellen für das körpereigene Immunsystem;
Einbringen von Alloantigenen (Fremd HLA Antigene, "Major Antigen") zur Aktivierung körpereigener T-Zellen und Auslösen einer Immunantwort gegen Tumorzellen mit mutiertem E-Cadherin "Minor Antigen");
Abtöten der Tumorzellen durch spezifisches Einbringen von Faktoren zur Induktion des Zelltodes (z.B. p53);
Konversion des malignen Phänotyps durch gezieltes Einbringen von Wildtyp Onkogenen/Suppressorgenen (z.B. E-Cadherin selbst); Aktivierung eines Protoxins in ein Toxin durch gezieltes Einschleusen eines Enzyms (z.B. Cytosin Deaminase, Umwandlung von 5-Fluorcytosin in das toxische 5-Fluoruracil);
- Ribozyme: z.B. gezielte Zerstörung der RNA für den Multidrug-Resistence Transporter

Weitere Beispiele von Anwendungsbereichen der E-Cadherin Mutationen
- Knochenmark Purching:: Spezifischer Nachweis von Tumorzellen in behandeltem Knochenmark in vitro und gleizeitige Verwendung des Antikörpers zur spezifischen Eliminierung von Tumorzellen aus dem Knochenmark (z.B. mittels eines am Antikörper gebundenen Toxins);
- Immuntherapie:: Beladung von dendritischen Zellen mit mutierten E-Cadherin-Peptidsequenzen (s. Tabelle 3) zur Antigenprasentation (Aktivierung von T-Zell-Klonen, die spezifisch gegen Tumorzellen gerichtet sind).

Beim intrazellulären Abbau von Proteinen entstehen Peptide mit unterschiedlicher Länge. Peptide mit einer Länge von 9-11 Aminosäuren werden vom MHC der Zelle "geprüft" (die Bindungsfähigkeit in der Peptid-Bindungsregion ist von einer bestimmten Anordnung der einzelnen Aminosäuren abhängig und unterscheidet zwischen "fremd" und "nicht fremd") und im Falle einer bestimmten Sequenz - die als fremd erkannt wurde - an der Zelloberfläche präsentiert. Es kann daraufhin, gefördert durch Kostimulatoren, eine Immunantwort erreicht werden. Bei den von uns erstmalig beschriebenen Peptidsequenzen des mutierten E-Cadherins und weiteren, durch Mutationen entstehenden Peptiden ist zu erwarten, daß diese teilweise vom MHC als fremdes Peptid präsentiert werden.
Die mangelnde Immunantwort im Patienten auf diesen Vorgang erklärt sich durch die Eigenschaft von Tumorzellen, Antigene schlecht zu präsentieren. Die gefunden mutationsüberspannenden Peptide können - in verschiedener Abwandlung der Länge - auf professionellen, antigenpräsentierenden Zellen (dentritrischen Zellen) aufgebracht werden. Durch das Vorhandensein aller notwendigen Kostimulatoren auf diesen Zellen kommt es dann zu einer entsprechenden T-Zell-Stimulation gegen solche MHC-Peptid-Komplexe. Auf diesem Wege werden dann auch die, zunächst vom Immunsystem ignorierten Tumorzellen als "fremd" erkannt und eliminiert.
Bei dieser Anwendung ist ein spezieller mutationsspezifischer Antikörper nicht nötig, wohl aber die Kenntnis über die neu entstandenen Peptidsequenzen, die erfindungsgemäß beschrieben wird.

Erfindungsgemäß werden die nachfolgenden Gegenstände und Verfahren mitumfaßt:

Monoklonaler Antikörper, der spezifisch gegen diejenigen Aminosäuresequenzen von mutiertem E-Cadherin gerichtet ist, die durch In-frame-Mutationen auf DNA-Ebene entstanden sind und
a. zum Verlust mindestens eines Basentripletts oder eines Multimeren hiervon in einem Exon auf RNA-Ebene und in der Folge zur Deletion mindestens einer Aminosäure des wt-E-Cadherin-Proteins führen, und/oder
b. zum Austausch von ein oder zwei Nukleotiden mindestens eines Basentripletts in einem Exon auf RNA-Ebene und in der Folge zum Austausch mindestens einer Aminosäure des wt-E-Cadherin-Proteins führen.

Monoklonaler Antikörper, der gegen diejenigen Aminosäuresequenzen von mutiertem E-Cadherin gerichtet ist, die durch In-frame-Mutationen auf DNA-Ebene entstanden sind und zum Verlust mindestens eines Basentripletts oder eines Multimeren hiervon in Exon 8, Exon 9 oder Exon 10 auf RNA-Ebene führen.

Monoklonaler Antikörper, der zumindest denjenigen Sequenz-Bereich aus einem oder mehreren der nachfolgenden Aminosäuresequenzen erkennt, der durch Deletion oder Aminosäureaustausch im Vergleich zu wt-E-Cadherin entstanden ist, ausgewählt aus mindestens einer Sequenz der nachfolgenden Gruppe:

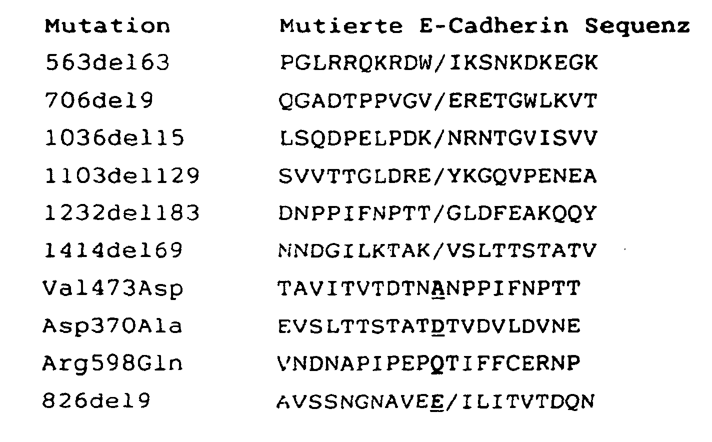

wobei "/" die Position einer Deletion und unterstrichene und fettgedruckte Buchstaben die durch Punktmutationen veränderten Aminosäuren anzeigen, je im Vergleich zum wt-E-Cadherin-Protein.

Die Erfindung umfaßt auch eine Mischung aus mindestens zwei der oben genannten monoklonalen Antikörper.

Die Erfindung umfaßt insbesondere monoklonale Antikörper, wie sie oben beschrieben wurden, die sich spezifisch gegen die Aminosäuresequenzen von mutiertem transmembranständigen-E-Cadherin richten.

In einer weiteren Ausführungsform der Erfindung wird ein Immuntest zum Nachweis von Magenkarzinomzellen umfaßt, der mindestens einen monoklonalen Antikörper der vorliegenden Erfindung mitumfaßt.

Hierin ist weiterhin Folgendes beschrieben:

Primer für PCR-Verfahren zur Amplifikation von DNA und cDNA-Sequenzen von mutierten Exon-Bereichen von E-Cadherin, die so ausgewählt sind, daß sie spezifisch die mutierten Sequenzen umfassen, die durch In-frame-Mutationen auf DNA-Ebene entstanden sind und
a. zum Verlust mindestens eines Basentripletts oder eines Multimeren hiervon in einem Exon auf RNA-Ebene und in der Folge zur Deletion mindestens einer Aminosäure des wt-E-Cadherin-Proteins führen, und/oder
b. zum Austausch von ein oder zwei Nukleotiden mindestens eines Basentripletts in einem Exon auf RNA-Ebene und in der Folge zum Austausch mindestns einer Aminosäure des wt-E-Cadherin-Proteins.

Primer für PCR-Verfahren zur Amplifikation von DNA und cDNA-Sequenzen von mutierten Exon-Bereichen von E-Cadherin, die so ausgewählt sind, daß sie spezifisch die mutierten Sequenzen einschließen, ausgewählt aus mindestns einem Primer der nachfolgenden Gruppe:

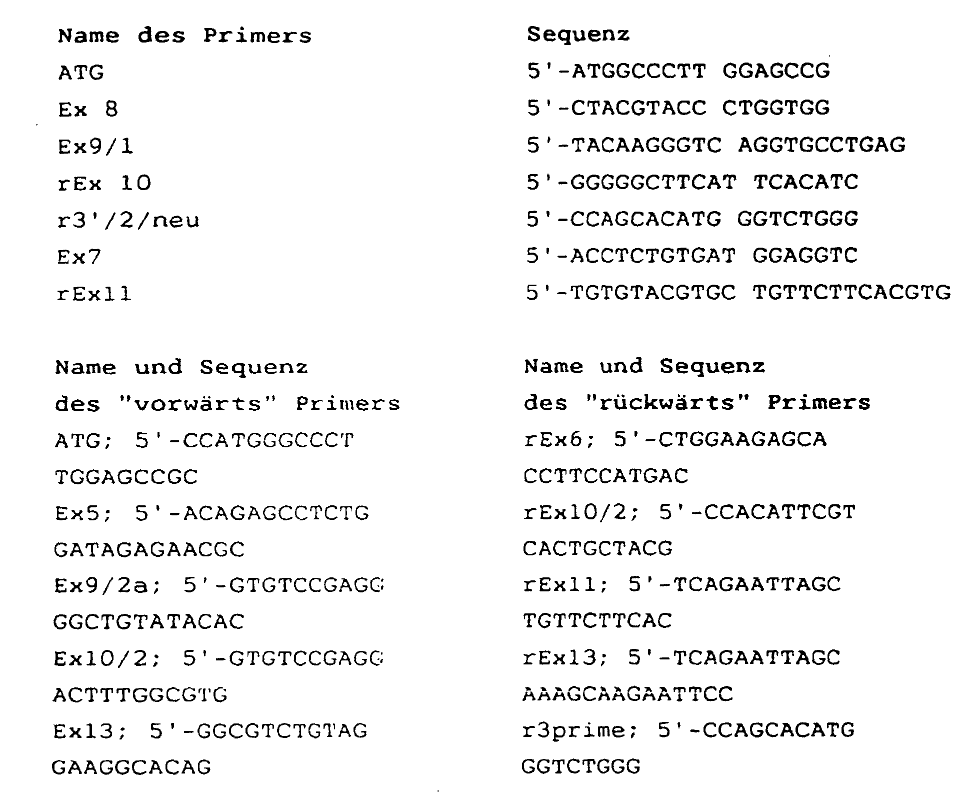

Auch wird ein therapeutisches oder diagnostisches Mittel gezeigt das als Wirksubstanz mindestens eine Nukleinsäure enthält, die spezifisch mit der DNA oder cDNA oder hiervon abgeleiteten RNA-Sequenzen von mutiertem E-Cadherin hybridisiert, wobei die DNA oder cDNA In-frame-Mutationen aufweist, die
a. zum Verlust mindestens eines Basentripletts oder eines Multimeren hiervon in einem Exon auf RNA-Ebene und in der Folge zur Deletion mindestens einer Aminosäure des wt-E-Cadherin-Proteins führen, und/oder
b. zum Austausch von ein oder zwei Nukleotiden mindestens eines Basentripletts in einem Exon auf RNA-Ebene und in der Folge zum Austausch mindestens einer Aminosäure des wt-E-Cadherin-Proteins führen.

Weiterhin beschrieben wird ein therapeutisches oder diagnostisches Mittel, das mindestns eine Nukleinsäure enthält, die zumindest mit einem Teil der nachfolgenden DNA-Sequenzen oder deren komplementären Strängen oder hiervon abgeleiteten RNA-Sequenzen zumindest unter stringenten Bedingungen hybridisiert, wobei zumindest der durch In-frame-Mutation entstandene Sequenzbereich miteingeschlossen ist:

Mutation 563del63:
CCT GGC CTC AGA AGA CAG AAG AGA GAC TGG / ATC AAA TCC AAC
AAA GAC AAA GAA GGC AAG

Mutation 706del9:
CAA GGA GCT GAC ACA CCC CCT GTT GGT GT / T GAA AGA GAA ACA
GGA TGG CTG AAG GTG ACA

Mutation 1036del15:
CTC AGC CAA GAT CCT GAG CTC CCT GAC AAA / AAC AGG AAC ACA
GGA GTC ATC AGT GTG GTC

Mutation 1103deldel129:
AGT GTG GTC ACC ACT GGG CTG GAC CGA GAG / TAC AAG GGT CAG
GTG CCT GAG AAC GAG GCT

Mutation 1232del183:
GAT AAT CCT CCG ATC TTC AAT CCC ACC ACG / GGC TTG GAT TTT
GAG GCC AAG CAG CAG TAC

Mutation 1414del69:
AAC AAC GAT GGC ATT TTG AAA ACA GCA AAG / TCT CTC ACC ACC
TCC ACA GCC ACC GTC

Mutation Asp370Ala:
ACA GCT GTG ATC ACA GTC ACT GAC ACC AAC GCT AAT CCT CCG ATC
TTC AAT CCC ACC ACG

Mutation Val473Asp:
GAG GTC TCT CTC ACC ACC TCC ACA GCC ACC GAC ACC GTG GAT GTG
CTG GAT GTG AAT GAA

Mutation Arg598Gln:
GTG AAT GAC AAC GCC CCC ATA CCA GAA CCT CAA ACT ATA TTC TTC
TGT GAG AGG AAT CCA

Mutation 826del9:
GCT GTG TCA TCC AAC GGG AAT GCA GTT GAG GA / G ATT TTG ATC
ACG GTA ACC GAT CAG AAT

Weiterhin werden therapeutische oder diagnostische Mittel gezeigt die als Wirksubstanz eine Nukleinsäure enthalten, die mit der oben beschriebenen Nukleinsäure unter stringenten Bedingungen hybridisiert.

Stringente Bedingungen im Sinne der vorliegenden Erfindung sind als solche Bedingungen definiert, die eine selektive und nachweisbare spezifische Bindung der Nukleinsäure an die erfindungsgemäß definierte Nukleinsäure ermöglichen. Eine derartige Hybridisierung unter stringenten Bedingungen bedeutet vorzugsweise, daß nach einer Hybridisierung bei 68°C in einer wässrigen Lösung oder bei 42°C in 50 % Formamid und anschließendem Waschen des Filters bei 65°C in einer wässrigen Lösung noch eine Bindung der Sonde an die erfindungsgemäß definierte Nukleinsäure oder die hiervon abgeleitete RNA nachweisbar ist. Falls notwendig können auch weniger drastische Hybridisierungs- und/oder Waschbedingungen angewandt werden.

Die erfindungsgemäß bereitgestellten monoklonalen Antikörper sind zur Diagnostik und Therapie von Magenkarzinomen, insbesondere vom diffusen Magenkarzinom, einsetzbar.

Zur Therapie können die monoklonalen Antikörper der vorliegenden Erfindung bspw. mit einem Mittel verbunden werden, das die Magenkarzinomzellen zumindest zum Teil selektiv eliminiert. Hierbei kann es sich bevorzugt um ein Toxin oder eine Strahlenquelle handeln.

Hierin gezeigt werden auch die näher bezeichneten DNA-Oligonukleotide und Oligopeptide. Diese sind zur Immuntherapie von Tumoren, insbesondere von Magenkarzinomen, einsetzbar.

Weiterhin (wird ein Verfahren zum Nachweis von Tumorzellen in einem menschliche Zellen enthaltenden Probenmaterial mit den nachfolgenden Schritten beschrieben:
a. Bereitstellen von menschliche Zellen enthaltendem Probenmaterial
b. Gewinnung der mRNA aus den menschlichen Zellen;
c. reverse Transkription der mRNA;
d. Durchführung einer Polymerasekettenreaktion unter Verwendung der Primer gemäß Anspruch 8 oder 9;
e. Auftrennen und Analysieren der Reaktionsprodukte der Polymerasekettenreaktion.

Auch Vektoren, die die Oligonukleotidsequenzen enthalten können bereit gestellt werden.

Erfindungsgemäß wurde somit erkannt, daß In-frame-Mutationen von E-Cadherin zur Diagnostik und Therapie von Magenkarzinomen einsetzbar sind. Der Ausdruck "In-frame-Mutation" bedeutet, daß auf der DNA-Ebene Mutationen oder Deletionen im E-Cadherin stattfinden, die auf RNA-Ebene zu einer Deletion oder zu einem Basenaustausch führen, wobei weder die Deletion noch die Mutation zu einer Verschiebung des Leserahmens führt.

### Methodik

### 1. Suche nach neuen E-Cadherin Mutationen

Gewebe von 63 Magenkarzinom-Patienten wurde untersucht. Frisches Tumorgewebe wurde nach Resektion in flüssigem Stickstoff tiefgefroren. Gesamt RNA von den tiefgefrorenen Gewebeproben wurde mittels Standardmethoden isoliert (Guanidinium Isothyiocyanat-Extraktion und CsCl Zentrifugation). Nach der reversen Transcription von zwei µg RNA wurde die gesamte kodierende Region der E-Cadherin cDNA mittels PCR amplifiziert. Die verwendeten PCR-Primer sind in der Tabelle 4 aufgelistet. Die Amplifikationsbedingungen für alle PCR Raktionen waren wie folgt: 1 Min Denaturierung bei 94°C, 1 Min Primer-Anlagerung bei 55°C, und 1 Min Elongation bei 72°C. Taq Polymerase and Amplifikationspuffer (1.5 mM MgCl₂) wurden von Perkin Elmer Corp., Foster City, CA, USA bezogen. Ein Biomed PCR-Gerät wurde verwendet (Biomed-Labordiagnostik, Oberschleissheim). Die Amplifikationsprodukte wurden durch Agarosegelelektrophorese überprüft und anschließend mit Glassmilch (GeanCleanII, Bio101 Inc., La Jolla, CA, USA) gereinigt. Die isolierten DNA-Amplifikate wurden anschließend in der gesamten Länge sequenziert (Sequenase Version 2.0 /USB, Cleveland, OH, USA).

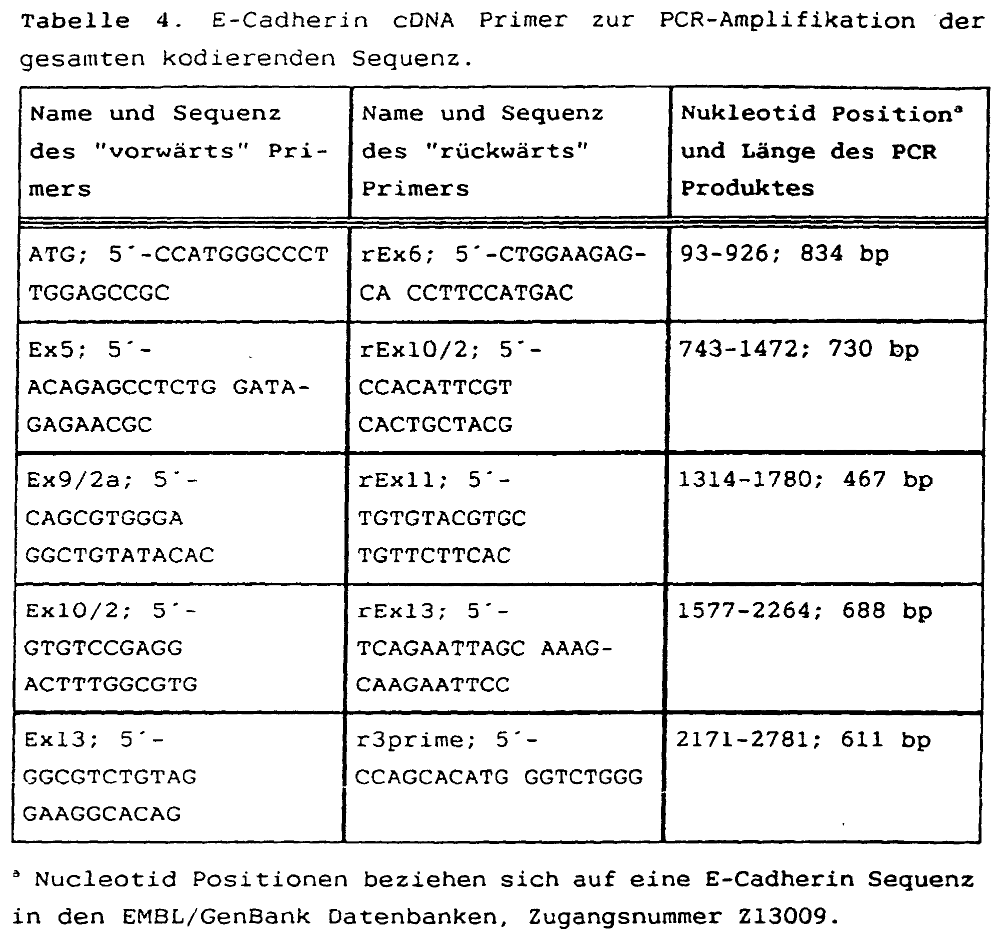

### 2. Immunhistochemische Untersuchung (nicht mutations-spezifisch):

Archivmaterial von Formalin fixiertem und Paraffin eingebettetem Gewebe wurde deparaffinisiert und rehydriert. Nach Zitronensäure- und Mikrowellenbehandlung wurden die Gewebsproben in 1% H₂O₂ für 15 Minuten inkubiert, um endogene Peroxidase zu blocken. Zum Nachweis einer E-Cadherin spezifischen Immunreaktivität wurden die Gewebe mit dem monoklonalen Antikörper HECD-1 (Takara Biomedicals, Japan, verdünnt auf 1:500) bei 4°C über 16 Stunden inkubiert Die Antikörperbindung wurde mit dem Avidin-Biotin-Complex (ABC) und der Peroxidase Methode (ABC Elite Kit, Vector, Burlingame, CA; Sigma FAST DAB, Sigma, Deisenhofen) sichtbar gemacht. Zur Kerngegenfärbung wurde Haemalaun benutzt. Sämtliche Tumorschnitte wiesen als Kontrolle auch Anteile nicht maligner Schleimhaut auf. Negativkontrollen wurden durch Ersatz des Antikörpers HECD-1 mit Phosphat gepuffertem NaCl durchgeführt.

### 3. Klonierung von E-Cadherin in Expressionsvektoren

Um die zelluläre Lokalisation von mutiertem E-Cadherin untersuchen zu können, wurden die mutierten Moleküle in Expressionsvektoren kloniert. Es wurden beispielhaft 4 verschiedene Mutanten der E-Cadherin mRNA sowie menschliches Wildtyp E-Cadherin in Expressionsvektoren einkloniert. Bei zwei dieser 4 Mutationen liegt ein kompletter Verlust eines Exons vor, entweder eine Deletion von Exon 8 (129 bp) oder eine Deletion von Exon 9 (183 bp). Eine weitere Mutation weist eine Teildeletion von Exon 10 (63 bp) auf. Bei der vierten Mutation handelt es sich um einen Basenaustausch in Exon 8, der eine potentielle Kalziumbindungsstelle zerstört. Alle zur Expressionsklonierung vorgesehenen Mutationen liegen im extrazellulären Bereich von E-Cadherin (für alle Mutationen s. Abb.1).

Als Ausgangspunkt für die Klonierung wurde Gesamt-RNA aus Frischmaterial von diffusen Magenkarzinomen, bei denen eine entsprechende Mutation identifiziert wurde (s.o.), benutzt. Die nach reverser Transkription entstandenen cDNAs des Wildtyp E-Cadherins sowie der Mutanten wurden jeweils in 2 Teilfragmenten (A+B) amplifiziert (s. Abb. 7). Die beiden Teilfragmente umfassen zusammen den gesamten kodierenden Bereich von E-Cadherin mit 2649 bp (Wildtyp). Die Amplifikation des Fragmentes A (5'-Bereich) erfolgte mit den Primern ATG und rEx10 (s. Tabelle 5). Für die Amplifikation des 3'-Fragmentes (B) der Wildtyp mRNA, der Exon 9 Deletion, der Teildeletion in Exon 10 und der Punktmutation in Exon 8 wurde das Primerpaar Ex8-r3'/2/neu eingesetzt. Die Amplifikation von Fragment B' der Exon 8 Deletionsmutante erfolgte mit dem Paar Ex 9/1-r3'/2/neu.
Die amplifizierten Teilfragmente A und B wurden jeweils in PCRII Vektoren (Invitrogen) einkloniert. Diese Vektoren ermöglichen über spezifische TA-Basenpaarung eine direkte und effektive Klonierung von PCR-Produkten. Über unterschiedliche Klonierungsstrategien, auf die hier nicht im Detail eingegangen werden kann, die aber im Bereich des Fachkönnens und -wissens des Fachmannes liegen, wurden die beiden cDNA Teilfragmente aller 5 Cadherin cDNAs (Wildtyp plus 4 Mutanten) entsprechend zusammenkloniert. Danach lagen alle 5 cDNA Konstrukte in PCRII Vektoren vor.

Um Klonierungsartefakte auszuschließen, die vor allem bei der Amplifikation (Taq-Fehler) entstanden sein konnten, wurden alle 5 cDNAs in der gesamten Länge durch Sequenzierung überprüft, einschließlich der Übergangsbereiche Vektor/Cadherin.

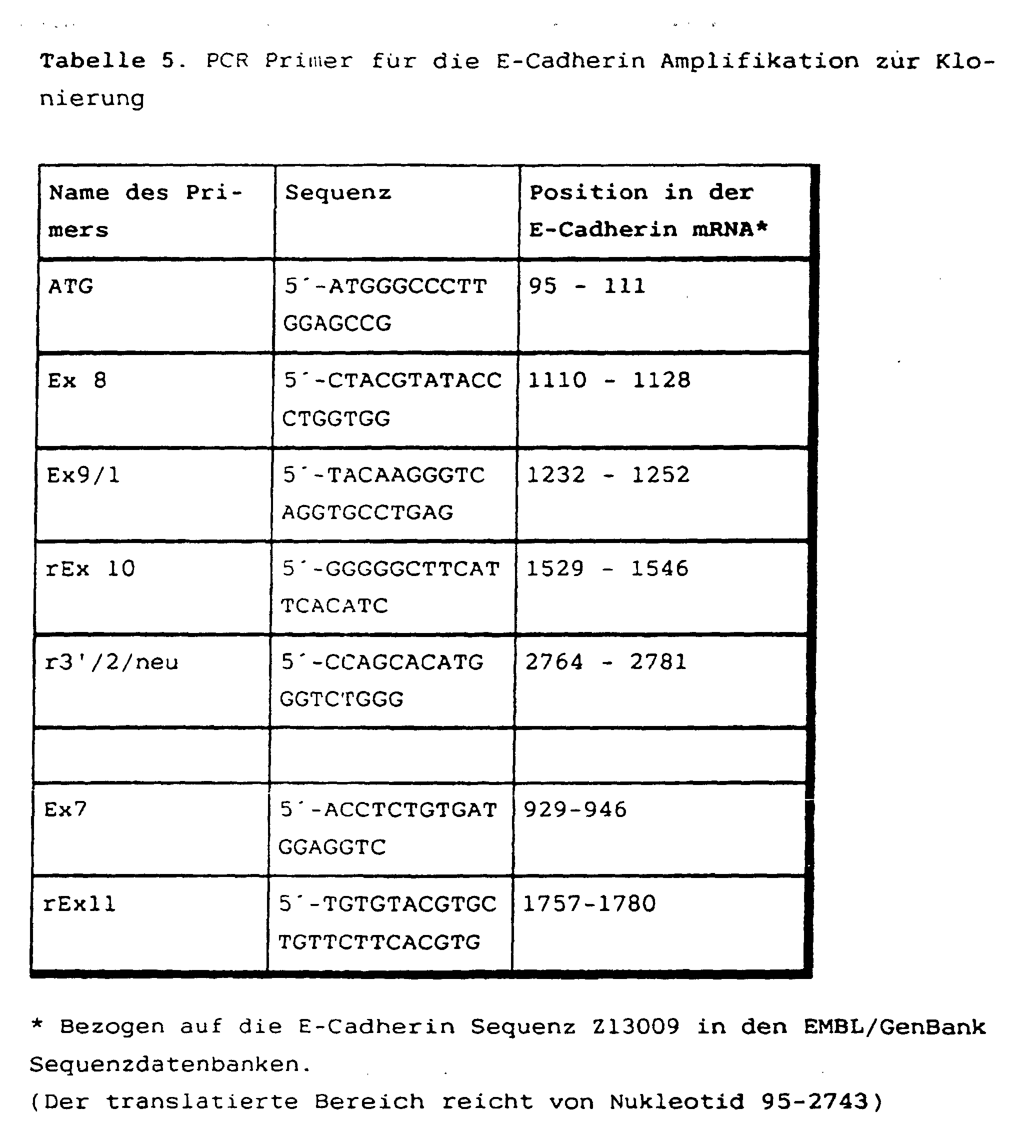

Die durch Sequenzierung abgesicherten Cadherin Konstrukte sind in einem weiteren Schritt in eukaryontische Expressionsvektoren einkloniert worden. Zum einen wurde der kommerziell erhältliche Vektor pBK-CMV (Stratagene) gewählt, der außer der Expression der gewünschten cDNA auch eine Selektion der exprimierenden Zellklone ermöglicht (Expression des Neomycin-Resistenzgens). Der zweite verwendete Vektor, pBAT, ist bereits erfolgreich bei Transfektionen von Wildyp E-Cadherin Konstrukten der Maus eingesetzt worden (Nose, A, Nagafuchi, A, Takeichi, M. Expressed recombinant cadherins mediate cell sorting in model systems. Cell 1988; 54: 993-1001).

### 4. Transiente Transfektionen und Nachweis von E-Cadherin mittels Western Blot, Immunfluoreszenz und Immunelektronenmikroskopie

Für die Transfektionen wurde die CaPO₄-Präzipitation etabliert. Die Transfektionseffizienz der Methode wurde anhand eines Kontrollplasmides pCMV (Stratagene) bestimmt. Der Vektor enthält das β-Galaktosidase Gen, wodurch Zellen, die den Vektor aufgenomen haben, nach Umsetzung des Farbstoffes X-Gal detektiert werden können (Blaufärbung). Die Effizienz der Methode lag bei 10%, für transiente Transfektionen ein sehr guter Wert.
In nachfolgenden Expressionsversuchen konnten nach Amplifikation der 5'-untranslatierten Region, die die Translationserkennungsregion (Kozak-Sequenz) enthielt, und Klonierung vor die cDNAs die so veränderten E-Cadherin cDNA Konstrukte in MDA-MB-435S Zellen, MIA PaCa-2, einer E-Cadherin negativen Pankreaszellinie, und in Neuro 2A (Neuroblastom)-zellen transient exprimiert werden.

Der Nachweis von E-Cadherin in den Zellkulturen erfolgte mittels Westernblot, Immunfluoreszenz und Immunelektronenmikroskopie.
Zur Herstellung eines Gesamtzellysates für den Western Blot wurde zuerst eine SDS-Lyse der Zellen (transfiziert oder untransfiziert) durchgeführt. Hier traten Unregelmäßigkeiten im Laufverhalten beim anschließenden Gellauf, der zur Überprüfung des Lysates nötig ist, auf. Alternativ wurde ein Zellextrakt mit Triton X-100 Lyse getestet. Die Überprüfung des Extraktes mit Polyacryamidgelelektrophorese und Coomassie Färbung zeigte hier einen effektiven Aufschluß der Zellen. Die Triton-Lyse wurde deshalb für alle weiteren Versuche benutzt.

Für den spezifischen Nachweis von E-Cadherin benutzten wir 4 verschiedene monoklonale Antikörper gegen E-Cadherin (HECD-1, Takara Biomedicals, Japan; AMST10, Saxon Biochemichals; DECMA-1, Sigma; ANTI-E-CADHERIN, Affinity Research Products Limited). Der Nachweis des Antigen-Antikörper-Komplexes im Westernblot erfolgte über einen zweiten, peroxidasegekoppelten Antikörper mittels Lumineszenzreaktion (ECL-Western, Amersham). Alle getesteten Antikörper zeigten eine spezifische, jedoch unterschiedlich starke Reaktion in einem Zellextrakt von einer E-Cadherin positiven Kontrollinie, A431 (epidermoides Karzinom, ATCC) und bei MDA-MB-435S-Zellen, die mit Wildtyp E-Cadherin transfiziert wurden. Bei den Extrakten der untransfizierten Linien MDA-MB-435S, Mia PaCa 2 und Neuro 2A konnte kein positives Signal detektiert werden.

Zur Durchführung der Immunfluoreszenz wurden auf Deckgläschen ausgesäte Zellen methanolfixiert und mit dem E-Cadherin spezifischen Antikörper HECD-1 markiert (erkennt sowohl Wildtyp wie auch mutiertes, z.B. Exon 9-deletiertes, E-Cadherin). Als Sekundärantikörper wurden Rhodamin (TRITC) gekoppelte Ziege-Anti-Maus-Antikörper (Dianova) verwendet.

Für den immunelektronenmikroskopischen Nachweis einer erfolreichen Transfektion wurden parallel nicht transfizierte MDA-MB-435S Zellen, mit mutiertem E-Cadherin transfizierte, sowie mit Wildtyp E-Cadherin transfizierte Zellen untersucht. Nach Fixierung und Inkubation mit einem goldmarkierten E-Cadherin-spezifischen Antikörper (HECD-1) wurden die markierten Zellen in Epon eingebettet und mit Uranylacetat/Bleicitrat kontrastiert. Bei untransfizierten MDA-MB 435S-Zellen konnte keine Markierung entdeckt werden, während die transfizierte Linien membran-assoziierte Gold-Markierungen zeigte, d.h. Wildtyp E-cadherin und auch mutiertes E-cadherin (z.B. mit einer Exon 9-Deletion, s. Abb. 3) waren in der Membran verankert.

### 5. Stabil E-Cadherin exprimierende Zellinien

Nachdem es uns gelungen war, sowohl Wildtyp wie auch mutiertes E-Cadherin in transient transfizierten Zellen eindeutig zu identifizieren, wurde die Herstellung von stabil E-Cadherin exprimierenden Zellen begonnen. Dies erfolgte durch Cotransfektion von MDA-MB-435S Zellen mit den jeweiligen pBAT Konstrukten (s."Klonierung") und einem pBAT Vektor ohne E-Cadherin cDNA, der dafür das Neomycin Resistenzgen enthielt. Es wurden drei Linien mit einer Deletion in Exon 9 (Del 9) des menschlichen E-Cadherins und drei Linien des menschlichen Wildtyp E-Cadherins (WT) etabliert. Die Überprüfung der mRNA durch RT-PCR der Del 9-Linien und der WT-Linien zeigte die erwarteten Fragmentgrößen (normal große mRNA für den Wildtyp (779 bp); verkürzte mRNA für die Del 9-Linien (596 bp), hier keine Wildtyp E-Cadherin mRNA Expression!). Die PCR Amplifikation wurde mit den Primern Ex7 und rEx11 durchgeführt (s. Tab.5). Im Western Blot mit Extrakten von stabil transfizierten Del 9-Linien und einer WT-Linie konnte eindeutig E-Cadherin Protein nachgewiesen werden.

Die Untersuchung der E-Cadherin Expression in der WT-Linie durch Immunfluoreszenz ergab die charakteristische Verteilung an den Kontaktstellen zur Nachbarzelle. Die Del-9 Linie zeigte mit dem HECD-1 Antikörper ebenfalls eine E-Cadherin Markierung an den Zell-zu-Zell Grenzen (Abb. 2).

### 6. RNA Schnellextraktion und nachfolgender PCR-Nachweis der Mutationen (am Beispiel einer Peritoneallavage)

Nach Abzentrifugieren der Zellen im Lavagepräparat und der RNA Extraktion (RNeasy Schnellextraktionskit, Quiagen) wird die mRNA revers transkribiert und die erhaltene E-Cadherin cDNA mittels PCR in der gesamten Länge amplifiziert (Primer und Bedingungen s. o.). Da es sich bei den E-Cadherin Mutationen hauptsächlich um in-frame Deletionen handelt, sind diese mittels Agarosegelelektrophorese rasch und sicher nachweisbar. Schon 9 Stunden nach Erhalt der Lavage sind somit mögliche E-Cadherin Deletionen, z.B. Exon 8- oder Exon 9-Verlust, und damit dissiminierte Tumorzellen, in der Bauchhöhle nachweisbar. Der klinische Entscheidungsverlauf kann aufgrund dieser Information direkt beeinflußt werden (z.B. zusätzliche intraperitoneale Therapien).
Hiermit ist eine hochspezifische Nachweismethode - selbst von wenigen Tumorzellen - sicher und rasch möglich. Verwechselungen von morphologisch "Tumor-ähnlichen" Zellen, z.B. entzündlich veränderte Mesothelzellen, sind durch die Tumorspezifität der E-Cadherin Mutationen ausgeschlossen.

### 7. Herstellung und Austestung eines mutations-spezifischen E-Cadherin Antikörpers (am Beispiel des Exon 9-Deletions-spezifischen Antikörpers 7E6).

Peptidsynthese. Das folgende Peptid wurde in einem Peptidsynthesizer Modell 430A (Applied Biosystems, Foster City,Cal., umgerüstet auf die Fast-Fmoc Modifikation) synthetisiert (Peptidsequenz: E-Cadherin Deletion von Exon 9, s. Tab. 3, 1232del183): Pro-Ile-Phe-Asn-Pro-Thr-Thr-Gly-Leu-Asp-Phe-Glu-Ala. Die Aminosäuren Asn, Thr, Asp und Glu wurden mit geschützten Seitenketten synthetisiert. Das Peptid wurde in 95%iger Trifluoressigsäure vom Syntheseharz gespalten, mit tert. Butylether gefällt und gefriergetrocknet. Das Rohprodukt wurde anschließend in 0,1%iger Trifluoressigsäure gelöst und auf einer Umkehrphasensäule (Aquapore 300A, C8, Applied Biosystems) mit einem linearen Gradienten zu 70%igem Acetonitril gereinigt. Die Reinheit des Peptids wurde in einem Massenspektrometer Modell AP100 (Perkin Elmer) mit Elektrospray Ionisation geprüft.

Koppelung an einen Carrier und Immunisierung: als Carrierprotein zur Immunisierung wurde keyhole limpet haemocyanin (KLH) aus Megathura crenulata verwendet (Calbiochem, Bad Soden) und in 0,5 M N-Methylimidazol Buffer (Sigma-Aldrich, Steinheim) pH 7,0 mittels 1-Ethyl-3-(dimethylamino-propyl)carbodiimid bei Raumtemperatur mit dem Peptid gekoppelt. Insgesamt wurden 2,8 mg Peptid an 2,0 mg Haemocyanin gekoppelt. Nach Dialyse gegen phosphatgepufferte Kochsalzlösung (PBS) wurde diese Lösung zur Immunisierung verwendet. Mit derselben Methode wurden 2 mg des Peptids an 2 mg 4x kristallisiertes Rinderserumalbumin (Behringwerke, Marburg) gekoppelt und zur Austestung der Hybridom-Kulturüberstände im ELISA Test verwendet. Die Immunisierung erfolgte in Lou/C Ratten. Dazu wurden 50µg KLH-gekoppeltes Peptid auf 500µl PBS verdünnt und mit gleicher Menge CFA emulsiert. Davon wurden je 500µl i.p. und s.c appliziert. Drei Monate später wurde mit KLH-gekoppeltes Peptid geboostet, jedoch ohne CFA. Die Fusion wurde 3 Tage später durchgeführt. Als Fusionslinie diente die murine Plamozytomlinie P3X63 Ag 8.653. Die Austestung erfogte im ELISA auf das spezifische Peptid und zur Kontrolle auf ein irrelevantes Peptid, das mit der identischen Kopplungschemie an BSA gekoppelt wurde. Hybridome, die nur mit dem spezifischen Peptid reagierten, wurden eingefroren und deren Überstände weiter analysiert. Die Subklassen wurden bestimmt mit subklassenspezifischen monoklonalen Antikörpern.
FACS Analyse: 200.000 MDA-MB-435S-Zellen, die mit Exon9 deletiertem E-Cadherin transfiziert wurden (s.o.) oder 200.000 nicht transfizierte MDA-MB-435S-Zellen wurden mit 50µl Hybridomüberstand inkubiert. Für die Detektion der gebundenen Antikörper wurden 50µl Ziege anti-Ratte-FITC in geeigneter Verdünnung für 30 min zugegeben. Die Zellen wurden im FACscan
(Becton) analysiert. Der mAb 7E6 hat die Subklasse "Ratte IgG1" und zeigte im FACscan die höchste Fluoreszenzintensität.
Immunfluoreszenz: Zur Durchführung der Immunfluoreszenz wurden auf Deckgläschen ausgesäte Zellen (entweder MDA-MB-435S-Zellen, die mit Exon 9 deletiertem E-Cadherin transfiziert wurden oder untransfizierte MDA-MB-435S-Zellen) methanolfixiert und mit dem E-Cadherin mutations-spezifischen Antikörper 7E6 markiert. Als Sekundärantikörper wurden FITC- gekoppelte Ziege-Anti-Ratte-Antikörper (Dianova) verwendet.

Western Blot: Zellextrakte von E-Cadherin transfizierten MDA-MB-435S-Zellen (entweder mit Wildtyp oder Exon 9-deletiertem E-Cadherin transfiziert) und weitere Zellinien wurden mit dem mutations-spezifischen Antikörper 7E6 im Western Blot untersucht. Bei den Extrakten der untransfizierten Linien MDA-MB-435S, Mia PaCa 2 (menschliches Pankreaskarzinom), Neuro 2A und A431 (epidermoides Karzinom, ATCC) konnte kein positives Signal mit dem Antikörper 7E6 detektiert werden. Die mit Wildtyp E-Cadherin transfizierten Zellen wiesen ebenfalls kein Signal mit 7E6 auf. Die einzige Zellinie, die mit dem mutations-spezifischen E-Cadherin Antikörper 7E6 reagiert hatte, war MDA-MB-435S, transfiziert mit Exon 9-deletiertem E-Cadherin.

Immunhistochemie: Archivmaterial (Formalin fixiertes und Paraffin eingebettetes Gewebe) von Magenkarzinom-Patienten mit molekularbiologisch nachgewiesenem E-Cadherin-Exon 9-Verlust wurde deparaffiniert und rehydriert. Nach Zitronensäure- und Mikrowellenbehandlung wurden die Gewebsproben in 1% H₂O₂ für 15 Minuten inkubiert, um endogene Peroxidase zu blocken. Zum Nachweis einer E-Cadherin mutations-spezifischen Immunreaktivität wurden die Gewebe mit dem monoklonalen Antikörper 7E6 bei 4°C über 16 Stunden inkubiert Die Antikörperbindung wurde mit dem Avidin-Biotin-Complex (ABC) und der Peroxidase Methode (ABC Elite Kit, Vector, Burlingame, CA; Sigma FAST DAB, Sigma, Deisenhofen) sichtbar gemacht. Zur Kerngegenfärbung wurde Haemalaun benutzt. Sämtliche Tumorschnitte wiesen als Kontrolle auch Anteile nicht maligner Schleimhaut auf. Negativkontrollen wurden durch Ersatz des Antikörpers 7E6 mit Phosphat gepuffertem NaCl durchgeführt.

## Patentansprüche

1. Monoklonaler Antikörper, der spezifisch gegen diejenigen Aminosäuresequenzen von mutiertem E-Cadherin gerichtet ist, die durch In-frame-Mutationen auf DNA-Ebene entstanden sind und
a. zum Verlust mindestens eines Basentripletts oder eines Multimeren hiervon in einem Exon auf RNA-Ebene und in der Folge zur Deletion mindestens einer Aminosäure des wt-E-Cadherin-Proteins führen, und/oder
b. zum Austausch von ein oder zwei Nukleotiden mindestens eines Basentripletts in einem Exon auf RNA-Ebene und in der Folge zum Austausch mindestens einer Aminosäure des wt-E-Cadherin-Proteins führen.

2. Monoklonaler Antikörper nach Anspruch 1,
**dadurch gekennzeichnet, daß**
er gegen diejenigen Aminosäuresequenzen von mutiertem E-Cadherin gerichtet ist, die durch In-frame-Mutationen auf DNA-Ebene entstanden sind und zum Verlust mindestens eines Basentripletts oder eines Multimeren hiervon in Exon 8, Exon 9 oder Exon 10 auf RNA-Ebene führen.

3. Monoklonaler Antikörper nach Anspruch 1 oder Anspruch 2,
**dadurch gekennzeichnet, daß**
er zumindest denjenigen Sequenz-Bereich aus einem oder mehreren der nachfolgenden Aminosäuresequenzen erkennt, der durch Deletion oder Aminosäureaustausch im Vergleich zu wt-E-Cadherin entstanden ist, ausgewählt aus mindestens einer Sequenz der nachfolgenden Gruppe: 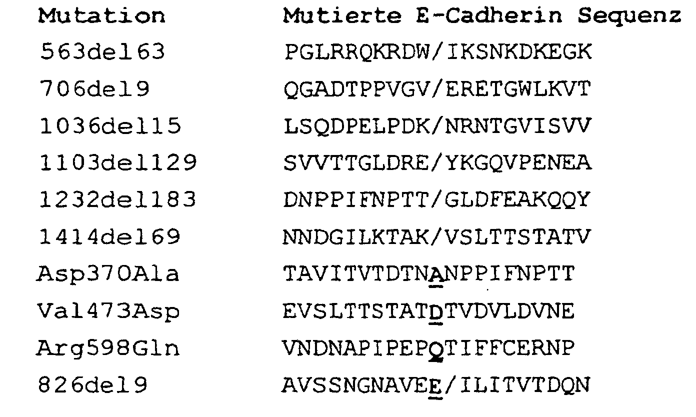 wobei "/" die Position einer Deletion und unterstrichene und fettgedruckte Buchstaben die durch Punktmutationen veränderten Aminosäuren anzeigen, je im Vergleich zum wt-E-Cadherin-Protein.

4. Mischung aus mindestens zwei monoklonalen Antikörpern, die gegen eine oder mehrere der obengenannten Aminosäuresequenzen gerichtet sind.

5. Monoklonaler Antikörper nach einem oder mehreren der Ansprüche 1 bis 4,
**dadurch gekennzeichnet, daß**
er spezifisch gegen die Aminosäuresequenzen von mutiertem transmembranständigen E-Cadherin gerichtet ist.

6. Monoklonale Antikörper nach einem oder mehreren der vorhergehenden Ansprüche produzierende Zellinie,
**dadurch gekennzeichnet, daß**
sie die Hybridomzellinie delta CAD-9, clone 7E6-1, Hinterlegungsnummer DSM ACC 2277, ist.

7. Immuntest zum Nachweis von Magenkarzinomzellen, enthaltend mindestens einen monoklonalen Antikörper nach einem oder mehreren der vorhergehenden Ansprüche.

8. Verwendung eines monoklonalen Antikörpers nach einem oder mehreren der vorhergehenden Ansprüche zur Herstellung eines Arzneimittels zur Diagnostik und Therapie von Magenkarzinomen.

9. Verwendung nach Anspruch 8,
**dadurch gekennzeichnet, daß**
der monoklonale Antikörper zur Therapie mit einem Mittel verbunden ist, das die Magenkarzinomzellen zum Teil selektiv eliminiert.

10. Verwendung nach Anspruch 9,
**dadurch gekennzeichnet, daß**
der monoklonale Antikörper mit einem Toxin oder einer Strahlenquelle gekoppelt ist.

## Claims

1. Monoclonal antibody specifically directed against amino acid sequences of mutated E-cadherin which have been generated by in-frame mutations on the DNA level and
a. lead to the loss of at least one base triplet or a multimer thereof in an exon on the RNA level and subsequently lead to the deletion of at least one amino acid of the wt E-cadherin protein, and/or
b. lead to the exchange of one or two nucleotides of at least one base triplet in an exon on the RNA level and subsequently to the exchange of at least one amino acid of the wt E-cadherin protein.

2. Monoclonal antibody according to claim 1,
**characterized in that**
said antibody is directed against those amino acid sequences of mutated E-cadherin which have been generated by in-frame mutations on the DNA level and lead to the loss of at least one base triplet or a multimer thereof in exon 8, exon 9, or exon 10 on the RNA level.

3. Monoclonal antibody according to claim 1 or claim 2,
**characterized in that**
said antibody at least recognizes the sequence region among one or more of the following amino acid sequences which has been generated by deletion or amino acid exchange as compared to wt E-cadherin, selected from at least one sequence of the following group: 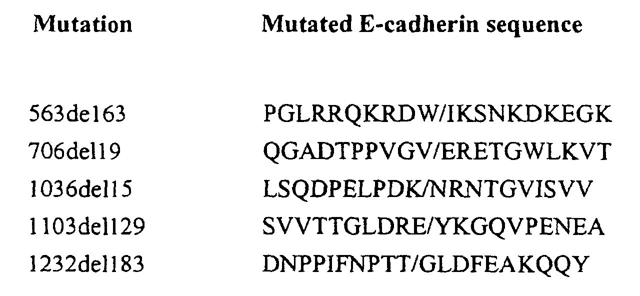 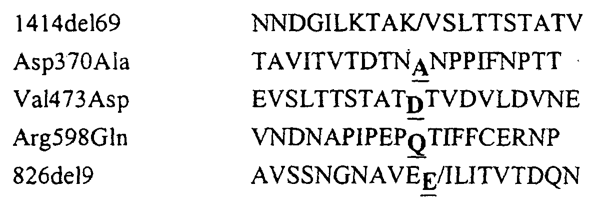 wherein "/" denotes the position of a deletion, and underlined and bold letters denote amino acids changed by point mutations, respectively, each in comparison to the wt E-cadherin protein.

4. Mixture of at least two monoclonal antibodies being specifically directed against one or more of the above mentioned amino acid sequences.

5. Monoclonal antibody according to one or more of claims 1 to 4,
**characterized in that**
said antibody is specifically directed against the amino acid sequences of mutated transmembrane E-cadherin.

6. Cell line producing monoclonal antibodies according to one or more of the preceding claims,
**characterized in that**
said cell line is the hybridoma cell line delta CAD-9, clone 7E6-1, deposit No. DSM ACC 2277.

7. Immune test for the detection of gastric carcinoma cells comprising at least one monoclonal antibody according to one or more of the preceding claims.

8. Use of a monoclonal antibody according to one or more of the preceding claims for the manufacture of a medicament for diagnosis and therapy of gastric carcinomas.

9. Use according to claim 8,
**characterized in that**
the monoclonal antibody for therapy is bound to a means for the selective elimination of some of the gastric carcinoma cells.

10. Use according to claim 9,
**characterized in that**
the monoclonal antibody is coupled to a toxin or a source of radiation.

## Revendications

1. Anticorps monoclonal qui est dirigé spécifiquement contre les séquences d'acides aminés de E-cadhérine mutée qui sont créées par mutation in-frame au plan de l'ADN et
a. qui provoquent la perte d'au moins un triplet de bases ou d'un multimère de celui-ci dans un exon au plan de l'ARN et ensuite la délétion d'au moins un acide aminé de la protéine wt-E-cadhérine et/ou
b. qui provoquent l'échange d'un ou de deux nucléotides d'au moins un triplet de bases dans un exon au plan de l'ARN et ensuite le remplacement d'au moins un acide aminé de la protéine wt-E-cadhérine.

2. Anticorps monoclonal suivant la revendication 1,
**caractérisé en ce que**
il est dirigé contre les séquences d'acides aminés de E-cadhérine mutée qui sont créées par des mutations in-frame au plan de l'ADN et qui provoquent la perte d'au moins un triplet de bases ou d'un multimère de celui-ci dans l'exon 8, l'exon 9 ou l'exon 10 au plan de l'ARN.

3. Anticorps monoclonal suivant la revendication 1 ou la revendication 2, **caractérisé en ce qu'**il reconnaît au moins la partie de séquence constituée d'une ou de plusieurs des séquences d'acides aminés suivantes, qui est créée par délétion ou remplacement d'acides aminés par rapport à la wt-E-cadhérine, choisie parmi au moins une séquence des groupes suivants : 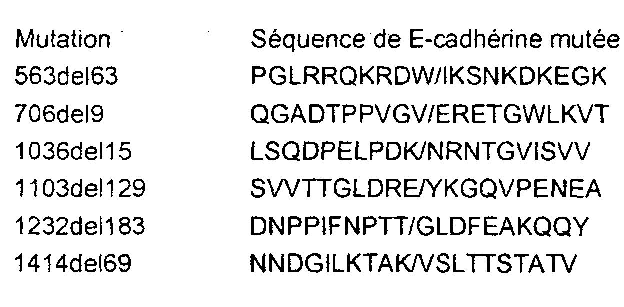 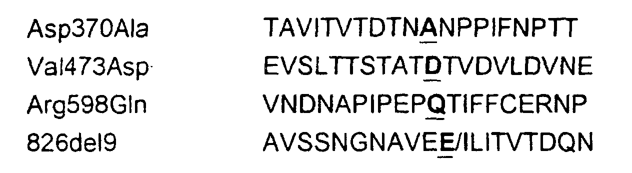 dans lesquelles « / » indique la position d'une délétion et les lettres soulignées et en gras les acides aminés modifiés par des mutations ponctuelles, respectivement, par rapport à la protéine wt-E-cadhérine.

4. Mélange d'au moins deux anticorps monoclonaux qui sont dirigés contre une ou plusieurs des séquences d'acides aminés mentionnées ci-dessus.

5. Anticorps monoclonal suivant l'une ou plusieurs des revendications 1 à 4, caractérisé en qu'il est dirigé spécifiquement contre les séquences d'acides aminés de E-cadhérine mutée de transmembrane.

6. Lignée cellulaire produisant des anticorps monoclonaux suivant l'une ou plusieurs des revendications précédentes, **caractérisée en ce que** c'est la lignée d'hybridome delta CAD-9, clone 7E6-1, numéro de dépôt DSM ACC 2277.

7. Test immunologique de détection de cellules de carcinome de l'estomac contenant au moins un anticorps monoclonal suivant l'une ou plusieurs des revendications précédentes.

8. Utilisation d'un anticorps monoclonal suivant l'une ou plusieurs des revendications précédentes pour la préparation d'un médicament de diagnostic et de thérapie de carcinomes de l'estomac.

9. Utilisation suivant la revendication 8, **caractérisée en ce que** l'anticorps monoclonal est relié pour la thérapie à un agent qui élimine sélectivement au moins en partie les cellules de carcinome de l'estomac.

10. Utilisation suivant la revendication 9, **caractérisée en ce que** l'anticorps monoclonal est couplé à une toxine ou à une source de rayonnement.
